# FASCICULE DE BREVET EUROPEEN

(11) **EP 3 060 545 B1**
(45) Date de publication et mention de la délivrance du brevet: **23.09.2020**
(21) Numéro de dépôt: 14824879.2
(22) Date de dépôt: 22.10.2014
(51) Int. Cl.: C07C 319/04

(54) **PROCÉDÉ DE SYNTHÈSE D'UN MERCAPTAN PAR ADDITION D'HYDROGÈNE SULFURÉ SUR UNE OLÉFINE**
VERFAHREN ZUR SYNTHESE EINES MERCAPTANS DURCH HINZUFÜGUNG VON WASSERSTOFFSULFID ZU EINEM OLEFIN
METHOD FOR SYNTHESISING A MERCAPTAN BY ADDING HYDROGEN SULPHIDE TO AN OLEFIN

(30) Priorité: 24.10.2013 FR 1360377
(43) Date de publication de la demande: 31.08.2016
(73) Titulaire: Arkema France, 92700 Colombes (FR)
(72) Inventeur: CAZAUX, Jean-Benoît, 64140 Billere (FR); OSTORERO, Delphine, 64230 Sauvagnon (FR)
(74) Mandataire: Arkema Patent
(86) Numéro de dépôt international: PCT/FR2014/052692
(87) Numéro de publication internationale: WO 2015/059412

(56) Documents cités:
- EP-A1- 0 354 460
- FR-A1- 2 844 794
- US-A- 3 257 302
- US-A- 6 133 496
- US-A1- 2006 247 475
- DATABASE WPI Week 198720 Thomson Scientific, London, GB; AN 1987-139159 XP002726930, & JP S62 77342 A (MITSUI PETROCHEM IND CO LTD) 9 avril 1987 (1987-04-09)

## Description

La présente invention concerne le domaine des mercaptans (encore appelés thiols) et a plus particulièrement pour objet un procédé de synthèse d'un mercaptan par addition catalytique d'hydrogène sulfuré sur une oléfine terminale.

L'invention concerne également une installation pour la mise en œuvre de ce procédé.

De nombreux travaux ont été effectués en vue de la mise au point de la préparation de ces composés, en raison de l'intérêt industriel des mercaptans ou thiols (cf. Forquy, Arretz, Heterogeneous Catalysis in Mercaptan Industrial Synthesis, Studies in Surface Science and Catalysis, Volume 41, (1988), pages 91-104)

On connaît par exemple un procédé largement employé, qui met en œuvre la réaction de thiolation des alcools par l'hydrogène sulfuré en présence d'un catalyseur de type alumine imprégnée par des oxydes métalliques, comme par exemple des oxydes de tungstène ou de césium ainsi que des dérivés alcalins. Cette thiolation est notamment mise en œuvre dans le cas de synthèse de mercaptans primaires.

On connait également le procédé, dans lequel l'addition d'hydrogène sulfuré est réalisée sur une oléfine. L'addition d'hydrogène sulfuré sur une oléfine est une réaction de sulfhydratation qui peut être orientée selon la règle de Markovnikov ou à l'inverse : anti-Markovnikov, selon que l'on se place en conditions catalytique ou radicalaire (photochimie par exemple). Des méthodes de sulfhydratation ont été proposées, visant à faire réagir sous pression, de l'hydrogène sulfuré (H₂S) sur une oléfine en présence de différents catalyseurs. Cette synthèse est très bien décrite dans la littérature (Reid, Organic chemistry of bivalent sulfur, vol I, 20, (1958)) et met en œuvre différents types de catalyseurs, tels que des catalyseurs acides homogènes (AlCl₃ tel que décrit dans le document US 2,531,601, ou encore BF₃ tel que décrit dans les documents GB 602,238, US 2,434,10 et US 2,443,852) ou hétérogènes présentant des sites acides forts (résines échangeuses de cations telles que décrites dans le document US 4,565,893, zéolites échangées telles que décrites dans le document US 4,102,931) ou encore de l'acide phosphorique supporté (US 2,950,324), de la silice avec de faibles quantités d'alumine (US 2,951,875), des alumines imprégnées ou pas avec des oxydes métalliques (US 6,162,952) ou des hétéropolyacides (US 3,036,133, FR 2 844 794).

L'article Thompson Scientific, London, AN 1987-139159, décrit la préparation de méthyl tert-butyl éther à partir d'isobutylène et de méthanol grâce à une installation comprenant un premier réacteur avec une première colonne de distillation et un second réacteur avec une seconde colonne de distillation.

Le document US 6,133,496 décrit une installation en ligne similaire à celle décrite ci-dessus avec un réacteur et une colonne de distillation suivis d'un deuxième réacteur et d'une seconde colonne de distillation pour isomériser des chaines hydrocarbonées saturées.

Le document US 2006/247475 décrit un procédé de préparation de thiols à partir d'alpha oléfines ayant au moins 5 atomes de carbone et d'hydrogène sulfuré en présence d'un catalyseur.

Le document US3257302 décrit une réaction en plusieurs étapes où l'hydrogène sulfuré est recyclé. Cependant, cette réaction est favorisée par les UV et n'est pas catalysée par une acide. le rendement de cette réaction d'addition catalytique d'hydrogène sulfuré sur une oléfine terminale. En effet, cette réaction d'addition conduit à un certain nombre de sous-produits, issus notamment des réactions anti-Markovnikov, des réactions d'isomérisation de la double liaison ainsi que de la réaction d'addition du mercaptan (formé dans la réaction principale) sur le réactif de départ, c'est-à-dire l'oléfine, conduisant à la formation de thioéthers.

Certaines de ces impuretés, telles que les oléfines internes, peuvent engendrer des contraintes significatives, telles qu'une accumulation de composés non réactifs sur le procédé et ne peuvent être purgées pour des raisons économiques évidentes.

Les performances de la viabilité d'un tel procédé sont compromises de manière importante par la présence de telles impuretés.

Les thioéthers obtenus en tant que sous-produits lors de la préparation de mercaptans n'ont généralement pas d'intérêt commercial et représentent une perte de rendement qu'il est toujours utile de limiter au maximum pour à la fois optimiser le rendement de production et le coût des étapes de purification.

Par conséquent, la Demanderesse cherche à améliorer ce procédé.

Des recherches ont été menées sur le catalyseur, afin d'améliorer sa sélectivité, de manière notamment à augmenter le rendement en thiol. Toutefois, la demanderesse n'a pas choisi ici de travailler sur le catalyseur, mais s'est orientée vers une amélioration du procédé en tant que tel.

Le problème technique a été résolu par l'ajout d'une étape de finition, qui suit l'étape d'addition de l'hydrogène sulfuré sur l'oléfine et une étape de recyclage des matières premières n'ayant pas réagi.

Par conséquent, l'invention porte sur un procédé de synthèse d'un mercaptan à partir d'une oléfine terminale et d'hydrogène sulfuré comportant au moins les étapes successives suivantes :
- A- addition catalytique d'hydrogène sulfuré sur une oléfine terminale, catalysée par un catalyseur acide, puis
- B- séparation des produits de la réaction d'addition en
   une fraction légère comprenant l'hydrogène sulfuré en excès et les oléfines, et
   en une fraction lourde comprenant au moins un mercaptan et éventuellement un ou plusieurs thioéthers,
- C- étape de finition par passage sur un catalyseur acide de la fraction légère issue de l'étape B, puis
- D- séparation des produits de l'étape de finition en
   une fraction riche en hydrogène sulfuré et
   en une fraction riche en produits d'addition puis
- E- recyclage de la fraction riche en hydrogène sulfuré vers l'étape d'addition catalytique A.

L'invention porte également sur une installation permettant de mettre en œuvre le procédé selon l'invention.

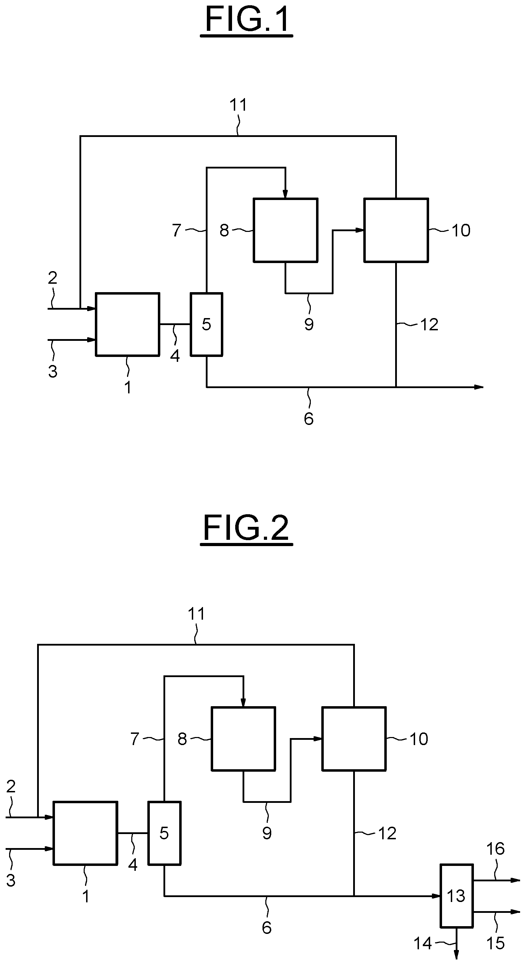

D'autres buts, caractéristiques et avantages de l'invention apparaîtront à la lecture de la description suivante et des figures 1 à 4 annexées, qui illustrent des modes de réalisation de l'installation selon l'invention et des exemples qui suivent.

D'autre part, tout intervalle de valeurs désigné par l'expression "entre a et b" représente le domaine de valeurs allant de plus de a à moins de b (c'est-à-dire bornes a et b exclues), tandis que tout intervalle de valeurs désigné par l'expression "de a à b" signifie le domaine de valeurs allant de a jusqu'à b (c'est-à-dire incluant les bornes a et b).

### Étape d'addition

Le procédé selon l'invention commence par une étape d'addition catalytique d'hydrogène sulfuré sur une oléfine terminale.

### Hydrogène sulfuré

L'hydrogène sulfuré est introduit dans le procédé en quantité suffisante pour obtenir la conversion de l'oléfine. En général, cette quantité peut correspondre à un rapport molaire H₂S/oléfine allant de 1 à 100, de préférence de 2 à 30, encore plus préférentiellement de 2 à 12. De préférence, l'hydrogène sulfuré est introduit en excès dans la réaction d'addition. Cette addition d'hydrogène sulfuré en excès permet de favoriser la réaction de formation du thiol par rapport aux réactions concurrentes de formation de thioéther ou d'oligomères.

De préférence, l'étape d'addition se fait sous pression de l'hydrogène sulfuré. De préférence, la pression va de 1MPa à 2MPa. Ces conditions permettent d'augmenter le niveau de conversion à quantité de catalyseur équivalent en jouant sur le paramètre du temps de séjour. Le procédé peut être mené de façon continue ou discontinue. De façon préférée, il est mené en continu.

La vitesse volumique horaire définie comme étant le ratio entre le débit volumique horaire d'oléfine et d'hydrogène sulfuré (dans les conditions de synthèse) sur le volume de catalyseur peut varier de manière importante selon l'activité du catalyseur. Cette valeur est généralement comprise entre 1 et 150 h⁻¹, de façon préférée entre 50 et 150 h⁻¹.

### Oléfine terminale

Par oléfine terminale, on entend au sens de la présente invention un hydrocarbure insaturé, dont la double liaison se situe nécessairement à l'une des extrémités de la chaîne hydrocarbonée.

De préférence, l'oléfine utilisée comme réactif de départ peut être de formule générale (1) suivante :

R₁R₂C=CH₂ (1),

dans laquelle R₁ désigne un atome d'hydrogène, et
R₂ représente un radical alkyle de 1 à 20 atomes de carbone, de préférence de 2 à 20 atomes de carbone, et plus particulièrement de 2 à 12 atomes de carbone, linéaire, ramifié ou cyclique. Le radical R₂ est éventuellement substitué par un groupement inerte vis-à-vis de la réaction considérée. Par exemple, mais de manière non restrictive, ce groupement peut être choisi parmi cycloalkyle, aryle, carboxy (-COOH), alkylcarbonyle (-C(O)OR), alcoxy (-OR), alkylcarbonyl-oxycarbonyle (-C(O)-O-C(O)-R), cyano (-CN), thiocarboxy (-C(S)-OH), mercaptocarbonyle (-C(O)-SH), alkoxy(thiocarbonyle) (-C(S)-OR), (alkylthio)carbonyle (-C(O)-SR), alkylcarbonylthiocarbonyle (-C(O)-S-C(O)-R), atome d'halogène, sulfo (-SO₃H), thionyle (R-S(O)-), alkylsulfonyle (R-S(O₂)-), alkoxysulfonyle (RO-S(O₂)-), radicaux comportant des fonctions silanes et/ou siloxanes et/ou phosphates.

R représente un radical alkyle de 1 à 20 atomes de carbone, de préférence de 2 à 20 atomes de carbone, et plus particulièrement de 2 à 12 atomes de carbone, linéaire, ramifié ou cyclique.

Les oléfines préférentiellement utilisées sont les oléfines linéaires terminales de C₃ à C₂₀. Les oléfines préférées sont choisies parmi le 1-butène, le 1-pentène, le 1-hexène, 1-heptène, le 1-octène, le 1-nonène, le 1-decène, 1-undecène et le 1-dodécène. Plus particulièrement, le 1-butène est utilisé. La réaction de sulfhydratation conduit dans ce cas au 2-butanethiol.

### Catalyseur

Le procédé selon l'invention utilise un catalyseur acide, de préférence un catalyseur acide solide, ce qui correspond à une réaction sous catalyse hétérogène.

De préférence, ces catalyseurs sont choisis parmi :
- un ou plusieurs hétéropolyacide(s) choisi (s) parmi :
   - (i) un composé de formules : H₃PW₁₂O₄₀,nH₂O, H₄SiWi₂O₄₀,nH₂O ou H₆P₂W₁₈O₆₂,nH₂O, dans lesquelles n est un nombre entier représentant le nombre de molécules d'eau de cristallisation, généralement compris entre 0 et 30, de préférence entre 6 et 20 ; et
   - (ii) un sel de potassium, rubidium, césium ou d'ammonium d'au moins un composé (i) ou un mélange de tels sels ;
- une zircone sulfatée,
- une zircone tungstée,
- une zéolithe,
- une résine cationique, et
- des oxydes métalliques.

L'hétéropolyacide (i) est généralement obtenu par la condensation de deux ou plus oxo-acides différents, tels que l'acide phosphorique, l'acide silicique ou l'acide tungstique. Il est soluble dans l'eau ou dans un solvant organique polaire. Le composé de formule : H₃PW₁₂O₄₀,nH₂O est connu sous la dénomination d'acide 12-phosphotungstique ou 12-tungstophosphorique, et est disponible dans le commerce. Le composé de formule : H₄SiW₁₂O₄₀,nH₂O est connu sous le nom d'acide 12-tungstosilicique ou 12-silicotungstique, et est également disponible dans le commerce.

Le composé de formule : H₆P₂W₁₈O₆₂,nH₂O peut être préparé selon le mode opératoire décrit dans la référence suivante : A. P. Ginsberg, Inorganic Synthesis, Vol 27, publié par J. Wiley & sons (1990) pages 105-107.

L'hétéropolyacide (ii) est un sel obtenu par substitution partielle d'un ou plusieurs protons de l'hétéropolyacide (i) par le cation correspondant. Il est clair pour l'homme du métier qu'une telle substitution ne peut être totale, sans quoi l'acidité serait perdue. Un tel sel est préparé a partir d'une solution de l'hétéropolyacide (i) à laquelle est ajoutée la quantité souhaitée du précurseur de l'alcalin ou de l'ammonium.

Le précurseur préféré est le chlorure ou le carbonate correspondant. Le sel précipité est séparé, puis séché dans des conditions douces, de préférence par centrifugation suivie d'une lyophilisation. On peut citer comme référence: N. Essayem, G. Coudurier, M. Fournier, J.C. Vedrine, Catal. Lett., 34 (1995) pages 224-225.

Ces hétéropolyacides peuvent être déposés sur des supports connus, tels que du carbone activé, de l'alumine, de la zircone, de la silice, de l'oxyde de thorium, de la pierre ponce, et des compositions silice-alumine.

La zircone sulfatée est préparée par imprégnation d'acide sulfurique sur un support d'oxyde de zirconium conformément au procédé décrit dans la référence: F. R. Chen, G. Coudurier, J-F Joly and J.C. Vedrin, J. Catal., 143 (1993) page 617.

La zircone tungstée est préparée par imprégnation d'oxyde de tungstène sur un support d'oxyde de zirconium, conformément au procédé décrit dans le brevet Soled et al. (US 5,113,034.)

Comme catalyseur à utiliser selon l'invention peuvent convenir également tous les différents polymères et copolymères à fonctions acide, connus dans l'art comme échangeurs de cations. En particulier, on peut employer des résines à base de polystyrène sulfoné réticulées, en particulier avec du divinylbenzène, des résines acryliques ou phénylacryliques à groupes carboxyliques libres, des résines du type phénol-formaldéhyde dérivées des acides phénol-sulfoniques, des échangeurs ligno-sulfoniques, etc... Des résines de ce genre se trouvent dans le commerce sous différentes dénominations, en particulier Allassion®, Cecacit®, Wofatites®, Levatites®, Imac®, Ionac®, Amberlites®, Amberlyst®, Liquorex®, Zeorex®, Zeocarb®, Dowex®, etc... Conviennent tout particulièrement les copolymères sulfonés du styrène avec le divinylbenzène, par exemple ceux que l'on trouve dans le commerce sous les dénominations Amberlyst®, Lewatit® ou Dowex® ; d'autre part, peuvent être employés avantageusement les copolymères de tétra-fluoroéthylène avec un acide perfluorosulfonique (en particulier l'acide perfluoro-3,6-dioxa-4-méthyl-7-octène sulfonique) connus sous la marque Nafion®. Quelle que soit la résine employée comme catalyseur, il faut veiller à ce qu'elle ne contienne pas plus de 0,5 % d'eau déterminable après 6 heures de séchage à 80°C (avantageusement moins de 0,2 % en poids d'eau).

Il est également possible d'utiliser des oxydes de molybdène, oxydes de cobalt, oxydes de chrome ou molybdate de cobalt sur alumine. Ces derniers peuvent être déposés sur des supports connus, tels que du carbone activé, de l'alumine, de la zircone, de la silice, de l'oxyde de thorium, de la pierre ponce, et des compositions silice-alumine.

De préférence, dans l'étape d'addition, un catalyseur de type acide de Lewis est utilisé. De préférence, un catalyseur de type acide de Bronsted est utilisé pour l'étape de finition.

De manière préférée, le catalyseur utilisé à l'étape d'addition est un catalyseur comportant des sites acides de Lewis.

De préférence, le catalyseur hétérogène comportant des sites acides de Lewis est choisi parmi les oxydes de molybdène, oxydes de cobalt, oxydes de chrome, molybdate de cobalt, les acides phosphotungstiques déposés sur des supports choisis parmi du carbone activé, de l'alumine, de la zircone, de la silice, de l'oxyde de thorium, de la pierre ponce et des compositions silice-alumine, et plus particulièrement de l'oxyde de chrome supporté sur alumine.

De préférence, le catalyseur utilisé pour l'étape de finition est différent du catalyseur utilisé sur la première réaction d'addition afin de maximiser la conversion.

Dans la première partie de la réaction, les réactifs décrits ci-dessus sont mis en contact, en présence d'une charge de la composition catalytique définie précédemment.

Ce procédé peut être effectué en phase gaz ou liquide ou en phase gaz / liquide. Dans un mode préféré, la réaction est conduite en phase gaz dans la mesure où les conditions de température et de pression utilisées sont telles que les réactifs et les produits sont à l'état gazeux.

On préfère mettre en œuvre le procédé dans un réacteur alimenté en continu par les réactifs, mais un réacteur de type batch peut également être utilisé.

La température de réaction varie selon l'oléfine utilisée et le degré de conversion désiré, mais se situe généralement dans un domaine allant de 30 à 350°C, de préférence de 100 à 350°C.

### Étape de séparation

Suit ensuite une étape de séparation des produits de la réaction d'addition en une fraction légère comprenant l'hydrogène sulfuré en excès et les oléfines terminales et/ou internes, et
en une fraction lourde comprenant au moins un mercaptan et éventuellement un ou plusieurs thioéthers.

La fraction destinée à l'étape de finition est la fraction légère. Elle comprend les réactifs n'ayant pas réagi : l'hydrogène sulfuré ajouté en excès dans le réacteur, l'oléfine terminale et l'oléfine isomérisée sous l'action du milieu réactionnel acide.

En effet, il a été observé, sous l'action du milieu acide, une migration de la double liaison terminale. Les oléfines obtenues sont appelées « oléfine interne » en raison de cette migration de la double liaison se situant à une des extrémités de la chaîne vers l'intérieur de la chaîne.

La seconde fraction à conserver comprend les produits de la réaction d'addition : les produits de l'addition selon Markovnikov majoritaires, les produits de l'addition anti-Markovnikov et des sulfures ou thioéthers.

De part les poids moléculaires de ces espèces dans le milieu réactionnel, la distillation peut être une méthode de séparation appropriée. Une séparation gaz/liquide est également possible.

### Étape de finition

La fraction légère issue de l'étape de séparation B, c'est-à-dire les réactifs non convertis en mercaptan ou en sulfures sur le réacteur d'addition sont traités sur un réacteur finisseur catalytique de technologie adaptée (lit fixe, multitubulaire, lit fluidisé, etc...), éventuellement en présence d'une quantité supplémentaire d'hydrogène sulfuré introduite directement dans le réacteur de finition.

Le procédé selon l'invention utilise un catalyseur acide, de préférence un catalyseur acide solide, ce qui correspond à une réaction sous catalyse hétérogène.

Ce réacteur finisseur met en œuvre un catalyseur acide. Ce catalyseur est un catalyseur acide présentant des sites acides forts. Plus particulièrement, il est choisi parmi les résines échangeuses de cations et les zéolites échangées ou non, et notamment les hétéropolyacide supporté, résine macroréticulée sulfonique de type Amberlyst® A15, A16, A35, A36, A70. Les résines Amberlyst® 35 et 36 dry sont notamment préférées. Ces résines sont fournies par la société DOW.

Dans l'étape de finition, le catalyseur est préférentiellement une résine macroréticulée sulfonique, comme par exemple celle commercialisée sous le nom « Amberlyst® 36 dry ».

Cette étape peut être effectuée à une température allant de 100 à 150°C dans la même gamme de pression d'hydrogène sulfuré que la première étape réactionnelle. Cette étape permet de convertir les oléfines isomérisées en les faisant réagir sur le catalyseur avec l'hydrogène sulfuré présent dans le réacteur.

Cette étape peut se faire en phase gaz ou en phase liquide, de façon continue ou discontinue (batch). De façon préférée, elle est menée de façon continue.

La vitesse volumique horaire définie comme étant le ratio entre le débit volumique horaire d'oléfine et d'hydrogène sulfuré (dans les conditions de synthèse) sur le volume de catalyseur peut varier de manière importante selon l'activité du catalyseur. Cette valeur va généralement de 1 à 150 h⁻¹, de façon préférée de 50 à 150 h⁻¹.

### Étape de séparation

Suit ensuite une étape de séparation des produits de l'étape de finition en une fraction riche en hydrogène sulfuré et
en une fraction riche en produits d'addition.

La fraction riche en produits d'addition comporte les produits de l'addition selon Markovnikov majoritaires, les produits de l'addition anti-Markovnikov et des sulfures.

### Recyclage

Les réactifs n'ayant pas réagi lors de l'étape de finition : l'hydrogène sulfuré ajouté en excès dans le réacteur d'addition et, éventuellement dans le réacteur de finition et les traces d'oléfines n'ayant toujours pas réagi même au cours de l'étape de finition sont recyclés vers le réacteur d'addition pour subir une nouvelle fois la réaction d'addition.

L'étape de recyclage peut comprendre une purge permettant d'éviter l'accumulation de sous-produits ne réagissant pas.

De préférence, le procédé décrit ci-dessus conduit à la synthèse de mercaptans secondaires.

Selon un premier mode de mise en œuvre, le procédé selon l'invention peut comprendre une étape de purification F complémentaire en fin de procédé.

La fraction lourde issue de l'étape de séparation B et la fraction riche en produits d'addition issue de l'étape de séparation E, comportant chacune au moins un mercaptan et éventuellement un ou plusieurs sulfures sont regroupées et purifiées afin d'isoler le ou les mercaptans attendus.

De part les poids moléculaires des espèces présentes dans le milieu réactionnel, la distillation peut être une méthode de séparation appropriée. Ainsi, cette étape F permet d'isoler le mercaptan secondaire, du mercaptan linéaire et des sulfures.

Selon un second mode de mise en œuvre, les sulfures issus de la dernière étape de purification F décrite ci-dessus, sont recyclés au niveau de l'étape de finition C.

La présence de ces sulfures permet de dissiper la chaleur de réaction de l'étape de finition. Elle permet également une réaction de sulfhydrolyse qui correspond à la réaction de l'hydrogène sulfuré sur un sulfure d'alkyle (préférentiellement ramifié), permettant de libérer deux molécules de mercaptan.

En effet, il est connu des documents US 4,927,972, US 4,059,636 et US 2006/0025633 que des sulfures ou thioéthers peuvent être convertis en mercaptan en présence d'hydrogène sulfuré et de catalyseurs, tels que des résines sulfoniques, des aluminosilicates, des acides 12-phosphotungstiques supportés sur alumine ou des compositions cobalt-molybdène sur alumine, ou encore des hétéropolyacides sur alumine.

Ainsi, en plus de la réaction d'addition de l'hydrogène sulfuré sur les oléfines internes et terminales présentes dans le milieu, la réaction de sulfhydrolyse est possible au sein de ce milieu réactionnel.

Par conséquent, cette étape de finition permet d'améliorer le rendement de la réaction en transformant une partie les produits secondaires de la réaction en mercaptan valorisable.

Ainsi, ce second mode de réalisation comprend les étapes successives suivantes :
- A- addition catalytique d'hydrogène sulfuré sur une oléfine terminale catalysée par un catalyseur acide, puis
- B- séparation des produits de la réaction d'addition en
   une fraction légère comprenant l'hydrogène sulfuré en excès et les oléfines, et
   en une fraction lourde comprenant au moins un mercaptan et éventuellement un ou plusieurs thioéthers
- C- étape de finition par passage sur un catalyseur acide de la fraction légère issue de l'étape B, puis
- D- séparation des produits de l'étape de finition en
   une fraction riche en hydrogène sulfuré et
   en une fraction riche en produits d'addition puis
- E- recyclage de la fraction riche en hydrogène sulfuré vers l'étape d'addition catalytique A,
- F- purification de la fraction lourde issue de l'étape de séparation B et la fraction riche en produits d'addition issue de l'étape de séparation E, réunies
   en une ou plusieurs fractions légères comportant un ou plusieurs mercaptans et
   en une fraction lourde comportant les sulfures puis
- G- recyclage de la fraction lourde de l'étape de purification précédente F vers l'étape de finition C.

Selon un troisième mode de mise en œuvre, les fractions comportant le ou les mercaptans et les sulfures issus de l'étape de séparation B et de l'étape de séparation E ne sont pas rassemblées. Selon ce mode de réalisation, une étape de purification est prévue pour chaque fraction.

Ainsi, ce troisième mode de réalisation comprend les étapes suivantes :
- A- addition catalytique d'hydrogène sulfuré sur une oléfine terminale catalysée par un catalyseur acide, puis
- B- séparation des produits de la réaction d'addition en
   une fraction légère comprenant l'hydrogène sulfuré en excès et les oléfines, et
   en une fraction lourde comprenant au moins un mercaptan et éventuellement un ou plusieurs thioéthers,
- C- étape de finition par passage sur un catalyseur acide de la fraction légère issue de l'étape B, puis
- D- séparation des produits de l'étape de finition en
   une fraction riche en hydrogène sulfuré et
   en une fraction riche en produits d'addition puis
- E- recyclage de la fraction riche en hydrogène sulfuré vers l'étape d'addition catalytique A.
- P1- purification de la fraction lourde issue de l'étape de séparation B
   en une ou plusieurs fractions légères comportant un ou plusieurs mercaptans et
   en une fraction lourde comportant les sulfures puis
- G1- recyclage de la fraction lourde de l'étape de purification précédente P1 vers l'étape de finition C,
- P2- purification de la fraction riche en produits d'addition issue de l'étape de séparation D,
en une ou plusieurs fractions légères comportant un ou plusieurs mercaptans et
en une fraction lourde comportant les sulfures.

### Synthèse du 2-butanethiol à partir du 1-butène

Il est à présent décrit la synthèse du 2-butanethiol à partir du 1-butène à titre de mode de mise en œuvre préféré du procédé selon l'invention.

Ainsi, le 1-butène conduit majoritairement au 2-butanethiol, également appelé sec-butylmercaptan (SBM), selon une addition de Markovnikov. L'appellation mercaptan secondaire provient du fait que l'atome de carbone porteur de la fonction thiol est un carbone secondaire.

Les sous produits qui peuvent être obtenus sont :
- le n-butylmercaptan (NBM), qui est le produit d'addition orienté selon une addition Anti-Markovnikov,
- les 2-cis-butène et 2-trans-butène, qui sont les produits d'isomérisation du 1-butène en milieu acide,
- les sulfures ou thioéthers, qui sont les produits de réaction entre le butène et le mercaptan.

Selon le mode opératoire et les conditions réactionnelles, les sous-produits seront plus ou moins obtenus.

Le schéma réactionnel ci-dessous illustre les espèces précitées :

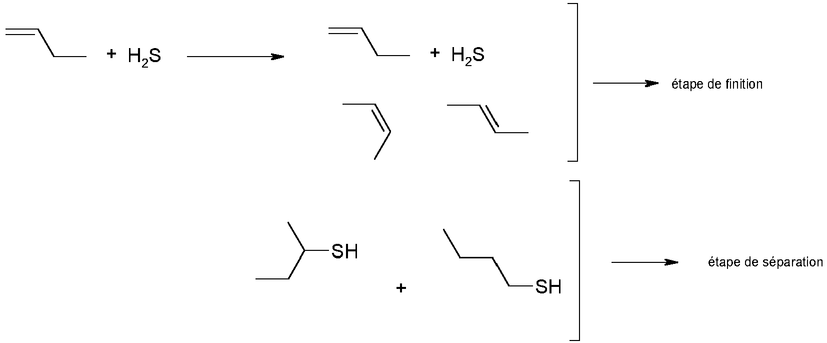

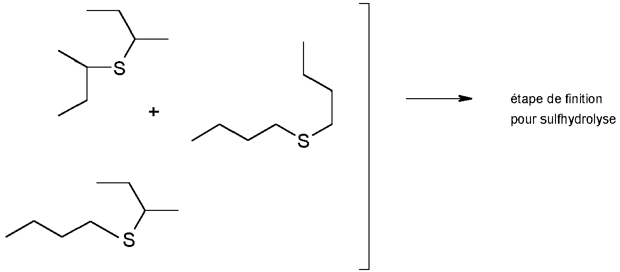

L'étape de finition présente dans le procédé selon l'invention a pour but de convertir les butènes-2 par réaction avec l'hydrogène sulfuré.

L'étape de recyclage des sulfures vers l'étape de finition en phase liquide a pour but de provoquer une réaction de sulfhydrolyse des sulfures conduisant à l'obtention de mercaptans.

Ainsi, ce procédé permet de transformer au maximum les espèces secondaires en mercaptans valorisables, conduisant ainsi à une augmentation du rendement.

L'invention porte également sur l'utilisation d'une installation mettant en œuvre le procédé décrit ci-dessus. Les détails de l'installation selon l'invention apparaîtront à la lecture de la description suivante donnée uniquement à titre d'exemple non limitatif et faite en référence aux figures 1 à 4 annexées qui illustrent les modes de réalisation de l'installation selon l'invention.

### Installation

*Figure 1*
   La figure 1 décrit une installation permettant la synthèse d'un mercaptan. L'installation comporte un réacteur d'addition (1) pour l'addition catalysée d'hydrogène sulfuré sur une oléfine terminale, alimentée en hydrogène sulfuré par une conduite (2), et en oléfine terminale par une conduite (3). La conduite (4), située en sortie du réacteur d'addition (1) alimente en flux de milieu réactionnel brut un premier dispositif de séparation (5).
   La conduite (6), raccordée en sortie du premier dispositif de séparation (5), permet d'évacuer la fraction lourde comprenant le ou les mercaptans et éventuellement un ou plusieurs thioéthers.
   La conduite (7), raccordée également en sortie du premier dispositif de séparation (5) récupère la fraction légère comprenant l'hydrogène sulfuré en excès et les oléfines. Cette conduite (7) alimente un réacteur de finition (8).
   La conduite (9) véhicule la totalité du milieu réactionnel du réacteur de finition (8) vers un second dispositif de séparation (10).
   Une conduite (11) située en sortie du second dispositif de séparation (10) recycle la fraction riche en hydrogène sulfuré vers le réacteur d'addition (1) ou vers la conduite (2) alimentant le réacteur d'addition (1) en matières premières. Cette conduite (11) permet ainsi un recyclage des réactifs n'ayant pas réagi, notamment de l'hydrogène sulfuré. Cette conduite (11) peut comporter une vanne permettant d'effectuer une purge de sous-produits s'accumulant dans le circuit.
   La canalisation (12) émanant du second dispositif de séparation (10) récupère la fraction riche en produits d'addition vers la canalisation (6) afin de regrouper les produits résultants de la réaction d'addition.
*Figure 2*
   La figure 2 est une variante de la figure 1. Les références de la figure 2 sont identiques à celles de la figure 1. La figure 2 comporte un dispositif de purification (13) supplémentaire.
   La canalisation (6) véhiculant la fraction lourde comprenant le ou les mercaptans et éventuellement un ou plusieurs thioéthers et raccordée en sortie du premier dispositif de séparation (5), raccordée à la canalisation (12) émanant du second dispositif de séparation (10) et récupérant la fraction riche en produits d'addition alimente le dispositif de purification (13). Ce dispositif de purification (13) permet d'isoler un mercaptan secondaire dans une première canalisation (16), un mercaptan linéaire dans une seconde canalisation (15) et évacuer une fraction lourde comportant les sulfures et autres sous-produits dans une troisième canalisation (14).
*Figure 3*

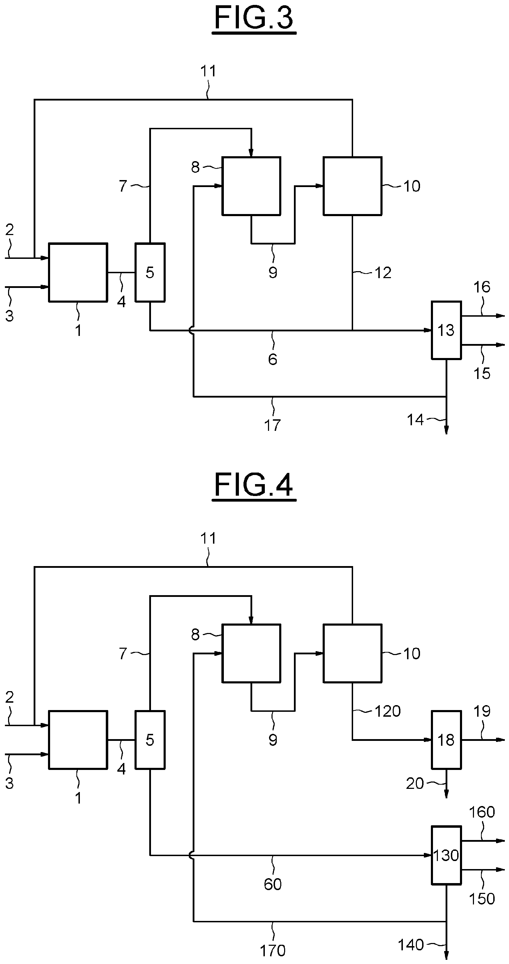

   La figure 3 est une variante de la figure 2. Les références de la figure 3 sont identiques à celles de la figure 2. La figure 3 comporte une canalisation (17) supplémentaire.
   La canalisation (17) véhiculant les sulfures et autres sous-produits est raccordée en sortie au dispositif de purification (13) et alimente le réacteur de finition (8).
*Figure 4*
   La figure 4 est une variante de la figure 1. Les références (1) à (5) et (7) à (11) de la figure 4 sont identiques à celles de la figure 1.

L'installation de la figure 4 comprend un premier dispositif de purification (130) raccordé en entrée au premier dispositif de séparation (5) via la conduite (60) et apte à isoler un mercaptan secondaire dans une première canalisation (160), un mercaptan linéaire dans une seconde canalisation (150) et des sulfures dans une troisième canalisation (140).

L'installation de la figure 4 comprend également une canalisation (170) véhiculant les sulfures et autres sous-produits raccordant en sortie le premier dispositif de purification (130) et en entrée le réacteur de finition (8).

L'installation de la figure 4 comprend enfin un second dispositif de purification (18) raccordé en entrée au second dispositif de séparation (10) via la conduite (120) et apte à isoler un mercaptan dans une première canalisation (19) et des sulfures dans une seconde canalisation (20).

Plus particulièrement, l'invention concerne une installation pour la mise en œuvre du procédé tel que défini précédemment, ladite installation comportant :
un réacteur d'addition (1), alimenté en hydrogène sulfuré et en oléfine par des conduites (2, 3),
une conduite (4), raccordée en sortie du réacteur d'addition (1) alimente un premier dispositif de séparation (5) en flux de milieu réactionnel brut,
une conduite (7), raccordée en sortie du premier dispositif de séparation (5) alimente un réacteur de finition (8) par la fraction légère comprenant l'hydrogène sulfuré en excès et les oléfines
une conduite (9), raccordée en sortie du réacteur de finition (8) alimente un second dispositif de séparation (10) en la totalité du milieu réactionnel du réacteur de finition (8),
une conduite (11) située en sortie du second dispositif de séparation (10) recycle la fraction riche en hydrogène sulfuré vers le réacteur d'addition (1) et
des conduites (12, 6, 120, 60) émanant des premier et second dispositifs de séparation (5, 10) récupèrent la fraction lourde comprenant au moins un mercaptan et éventuellement un ou plusieurs thioéthers et la fraction riche en produits d'addition, en les regroupant dans une seule conduite.

Les exemples suivants sont donnés à titre illustratif de la présente invention.

### Exemples de fonctionnement au laboratoire de l'addition catalytique de sulfure d'hydrogène sur une oléfine terminale.

### Exemple 1 ; procédé selon l'invention

L'étape d'addition d'hydrogène sulfuré sur un butène-1 se fait dans les conditions suivantes :

### 1- Premier réacteur catalytique en lit fixe chargé avec 200 cm³ de catalyseur (19% Cr₂O₃ / Alumine) :

Butène-1 : 75 g/h
H₂S : 92 NL/h, correspondant à un ratio molaire H₂S/butène = 2
T moyenne sur le lit catalytique = 245°C
P = 1,4 MPa
VVH = 60 h⁻¹

Les résultats obtenus pour cette étape d'addition sont les suivants :
Conversion : 83%
Sélectivités : 81/14/5 (SBM / NBM / thioéthers)

Les butènes non convertis ont alors la composition molaire suivante : 10% butène-1 / 45% butène-2 cis / 45% butène-2 trans

La fraction de butènes non convertis passe dans le réacteur finisseur selon les conditions suivantes :

### 2- Réacteur finisseur en lit fixe chargé avec 100 cm³ de résine sulfonique A36 dry (DOW)

Butènes : 7,3 NL/h
H₂S : 120 NL/h
VVH = 120 h⁻¹
Pas de solvant.

Les résultats obtenus pour cette étape de finition sont les suivants :
Conversion : 97,5%
Sélectivités : 87% SBM, 13% thioéthers

Ces deux étapes conduisent de ce fait à une conversion de 99,5% de butène-1 et une sélectivité suivante :
SBM : 81,6%
NBM : 11,6%
Sulfures : 6,2%
avec une alimentation en butènes de : 99,5% butène-1/ 0,5% butènes-2.

### Exemple 2 : Procédé comparatif

La première étape du procédé est identique à celle de l'exemple 1.

### 1-Réacteur catalytique en lit fixe chargé avec 200 cm³ de catalyseur (19% Cr₂O₃ / Alumine) :

Butène-1 : 75g/h
H₂S : 92 NL/h correspondant à un ratio molaire H₂S/butène = 2
T moyenne sur le lit catalytique = 245°C
P = 1,4 MPa
VVH =60 h⁻¹

Performances :
Conversion 83%
Sélectivités : 81/14/5 (SBM / NBM / thioéthers)

Les butènes non convertis ont alors la composition molaire suivante : 10% butène-1 / 45% butène-2 cis / 45% butène-2 trans

La fraction de butènes non convertis est recyclée au niveau du réacteur catalytique selon les conditions suivantes :
Alimentation butènes : 75 g/h avec 20% butènes-2 dans le butène-1
H₂S : 92 NL/h correspondant à un ratio molaire H₂S/butène = 2
T moyenne sur le lit catalytique = 245°C
P = 1,4 MPa
VVH =80 h⁻¹

Ces étapes d'addition et de recyclage conduisent à une conversion de 60% et une sélectivité de 82/12/6 (SBM/NBM/thioéthers).

Les butènes-2, ne réagissant pas, entrainent une chute de productivité et s'accumulent dans le recyclage finissant par rendre le procédé inexploitable.

## Revendications

1. Procédé de synthèse d'un mercaptan à partir d'une oléfine terminale et d'hydrogène sulfuré comportant au moins les étapes successives suivantes :
- A- addition catalytique d'hydrogène sulfuré sur une oléfine terminale catalysée par un catalyseur acide, puis
- B- séparation des produits de la réaction d'addition en
une fraction légère comprenant l'hydrogène sulfuré en excès et les oléfines, et
en une fraction lourde comprenant au moins un mercaptan et éventuellement un ou plusieurs thioéthers,
- C- étape de finition par passage sur un catalyseur acide de la fraction légère issue de l'étape B, puis
- D- séparation des produits de l'étape de finition en une fraction riche en hydrogène sulfuré et
en une fraction riche en produits d'addition puis
- E- recyclage de la fraction riche en hydrogène sulfuré vers l'étape d'addition catalytique A.

2. Procédé selon la revendication 1, **caractérisé en ce que** le mercaptan synthétisé est un mercaptan secondaire.

3. Procédé selon la revendication 1 ou 2, **caractérisé en ce que** le catalyseur utilisé à l'étape d'addition est un catalyseur comportant des sites acides de Lewis.

4. Procédé selon l'une quelconque des revendications précédentes, **caractérisé en ce que** la réaction d'addition à l'étape A et/ou l'étape de finition C sont des catalyses hétérogènes mettant en œuvre un catalyseur acide solide.

5. Procédé selon la revendication 3 ou 4, **caractérisé en ce que** le catalyseur comportant des sites acides de Lewis est choisi parmi les oxydes de molybdène, oxydes de cobalt, oxydes de chrome, molybdate de cobalt, les acides phosphotungstiques déposés sur des supports choisis parmi du carbone activé, de l'alumine, du zircone, de la silice, de l'oxyde de thorium, de la pierre ponce et des compositions silice-alumine, de préférence de l'oxyde de chrome supporté sur alumine.

6. Procédé selon l'une quelconque des revendications précédentes, **caractérisé en ce que** l'oléfine terminale est de formule générale (1) suivante :
R₁R₂C=CH₂ (1),
dans laquelle R₁ désigne un atome d'hydrogène, et
R₂ représente un radical alkyle de 1 à 20 atomes de carbone, de préférence de 2 à 20 atomes de carbone, et plus particulièrement de 2 à 12 atomes de carbone, linéaire, ramifié ou cyclique.

7. Procédé selon la revendication 6, **caractérisé en ce que** l'oléfine terminale est le 1-butène.

8. Procédé selon l'une quelconque des revendications précédentes, **caractérisé en ce que** le catalyseur utilisé pour l'étape de finition est un catalyseur acide présentant des sites acides forts, de préférence choisi parmi les résines échangeuses de cations et les zéolites échangées ou non.

9. Procédé selon l'une quelconque des revendications précédentes, **caractérisé en ce qu'**il comprend l'étape supplémentaire suivante :
- F- purification de la fraction lourde issue de l'étape de séparation B et la fraction riche en produits d'addition issue de l'étape de séparation E, réunies
en une ou plusieurs fractions légères comportant un ou plusieurs mercaptans et
en une fraction lourde comportant les sulfures.

10. Procédé selon l'une quelconque des revendications précédentes, **caractérisé en ce qu'**il comprend l'étape supplémentaire suivante :
- G- recyclage de la fraction lourde de l'étape de purification F définie à la revendication 9 vers l'étape de finition C.

11. Utilisation d'une installation pour la mise en œuvre d'un procédé de synthèse d'un mercaptan à partir d'une oléfine terminale et d'hydrogène sulfuré tel que défini à l'une quelconque des revendications précédentes comportant :
un réacteur d'addition (1), alimenté en hydrogène sulfuré et en oléfine par des conduites (2, 3),
une conduite (4), raccordée en sortie du réacteur d'addition (1) alimente un premier dispositif de séparation (5) en flux de milieu réactionnel brut,
une conduite (7), raccordée en sortie du premier dispositif de séparation (5) alimente un réacteur de finition (8) par la fraction légère comprenant l'hydrogène sulfuré en excès et les oléfines
une conduite (9), raccordée en sortie du réacteur de finition (8) alimente un second dispositif de séparation (10) en la totalité du milieu réactionnel du réacteur de finition (8),
une conduite (11) située en sortie du second dispositif de séparation (10) recycle la fraction riche en hydrogène sulfuré vers le réacteur d'addition (1) et
des conduites (12, 6, 120, 60) émanant des premier et second dispositifs de séparation (5, 10) récupèrent la fraction lourde comprenant au moins un mercaptan et éventuellement un ou plusieurs thioéthers et la fraction riche en produits d'addition, en les regroupant dans une seule conduite.

12. Utilisation d'une installation selon la revendication 11, **caractérisée en ce qu'**elle comprend un dispositif de purification (13), raccordé aux conduites (6, 12) émanant des premier et second dispositifs de séparation (5, 10), le dispositif de purification (13) étant apte à isoler un mercaptan secondaire dans une première canalisation (16), un mercaptan linéaire dans une seconde canalisation (15) et évacuer une fraction lourde comportant les sulfures et autres sous-produits dans une troisième canalisation (14).

13. Utilisation d'une installation selon la revendication 12, **caractérisée en ce qu'**elle comprend une canalisation (17), raccordée en sortie au dispositif de purification (13), véhiculant les sulfures et autres sous-produits, la canalisation (17) alimentant le réacteur de finition (8).

## Patentansprüche

1. Verfahren zur Synthese eines Mercaptans aus einem endständigen Olefin und Schwefelwasserstoff, das mindestens die folgenden aufeinanderfolgenden Schritte umfasst:
- A- durch einen sauren Katalysator katalysierte katalytische Addition von Schwefelwasserstoff an ein endständiges Olefin, dann
- B- Trennung der Produkte der Additionsreaktion in eine leichte Fraktion, die überschüssigen Schwefelwasserstoff und Olefine umfasst, und eine schwere Fraktion, die mindestens ein Mercaptan und gegebenenfalls einen oder mehrere Thioether umfasst,
- C- Fertigstellungsschritt durch Führen der leichten Fraktion aus Schritt B über einen sauren Katalysator, dann
- D- Trennung der Produkte des Fertigstellungsschritts in
eine schwefelwasserstoffreiche Fraktion und
eine an Additionsprodukten reiche Fraktion, dann
- E- Zurückführen der schwefelwasserstoffreichen Fraktion zum Schritt der katalytischen Addition A.

2. Verfahren nach Anspruch 1, **dadurch gekennzeichnet, dass** es sich bei dem synthetisierten Mercaptan um ein sekundäres Mercaptan handelt.

3. Verfahren nach Anspruch 1 oder 2, **dadurch gekennzeichnet, dass** es sich bei dem im Additionsschritt verwendeten Katalysator um einen Katalysator mit Lewis-sauren Zentren handelt.

4. Verfahren nach einem der vorhergehenden Ansprüche, **dadurch gekennzeichnet, dass** es sich bei der Additionsreaktion in Schritt A und/oder dem Fertigstellungsschritt C um heterogene Katalysen unter Verwendung eines festen sauren Katalysators handelt.

5. Verfahren nach Anspruch 3 oder 4, **dadurch gekennzeichnet, dass** der Katalysator mit Lewis-sauren Zentren aus Molybdänoxiden, Cobaltoxiden, Chromoxiden, Cobaltmolybdat und Wolframatophosphorsäuren, die auf Trägern, die aus Aktivkohle, Aluminiumoxid, Zirconiumdioxid, Siliciumdioxid, Thoriumoxid, Bimsstein und Siliciumdioxid-Aluminiumoxid-Zusammensetzungen ausgewählt sind, abgeschieden sind, vorzugsweise auf Aluminiumoxid geträgertem Chromoxid, ausgewählt wird.

6. Verfahren nach einem der vorhergehenden Ansprüche, **dadurch gekennzeichnet, dass** das endständige Olefin die folgende allgemeine Formel (1) aufweist:
R₁R₂C=CH₂ (1),
in der R₁ für ein Wasserstoffatom steht und
R₂ für einen linearen, verzweigten oder cyclischen Alkylrest mit 1 bis 20 Kohlenstoffatomen, vorzugsweise 2 bis 20 Kohlenstoffatomen und spezieller 2 bis 12 Kohlenstoffatomen steht.

7. Verfahren nach Anspruch 6, **dadurch gekennzeichnet, dass** es sich bei dem endständigen Olefin um 1-Buten handelt.

8. Verfahren nach einem der vorhergehenden Ansprüche, **dadurch gekennzeichnet, dass** es sich bei dem für den Fertigstellungsschritt verwendeten Katalysator um einen sauren Katalysator mit stark sauren Zentren handelt, der vorzugsweise aus Kationenaustauscherharzen und ausgetauschten oder nicht ausgetauschten Zeolithen ausgewählt ist.

9. Verfahren nach einem der vorhergehenden Ansprüche, **dadurch gekennzeichnet, dass** es den folgenden zusätzlichen Schritt umfasst:
- F- Reinigung der schweren Fraktion aus dem Trennungsschritt B und der an Additionsprodukten reichen Fraktion aus dem Trennungsschritt E nach Vereinigung
in eine oder mehrere leichte Fraktionen, die ein oder mehrere Mercaptane umfassen, und
in eine schwere Fraktion, die Sulfide umfasst.

10. Verfahren nach einem der vorhergehenden Ansprüche, **dadurch gekennzeichnet, dass** es den folgenden zusätzlichen Schritt umfasst:
- G- Zurückführen der schweren Fraktion aus dem Reinigungsschritt F gemäß Anspruch 9 in den Fertigstellungsschritt C.

11. Verwendung einer Anlage zur Durchführung eines Verfahrens zur Synthese eines Mercaptans aus einem endständigen Olefin und Schwefelwasserstoff gemäß einem der vorhergehenden Ansprüche, umfassend:
einen Additionsreaktor (1), der über die Leitungen (2, 3) mit Schwefelwasserstoff und Olefin gespeist wird,
eine mit dem Ausgang des Additionsreaktors (1) verbundene Leitung (4), die eine erste Trennvorrichtung (5) mit einem Strom von rohem Reaktionsmedium speist,
eine mit dem Ausgang der ersten Trennvorrichtung (5) verbundene Leitung (7), die einen Fertigstellungsreaktor (8) mit der leichten Fraktion, die überschüssigen Schwefelwasserstoff und Olefine umfasst, speist,
eine mit dem Ausgang des Fertigstellungsreaktors (8) verbundene Leitung (9), die eine zweite Trennvorrichtung (10) mit der Gesamtheit der Reaktionsmischung aus dem Fertigstellungsreaktor (8) speist,
eine am Ausgang der zweiten Trennvorrichtung (10) angeordnete Leitung (11), die die schwefelwasserstoffreiche Fraktion zum Additionsreaktor (1) zurückführt, und
von der ersten und zweiten Trennvorrichtung (5, 10) kommende Leitungen (12, 6, 120, 60), die die schwere Fraktion, die mindestens ein Mercaptan und gegebenenfalls einen oder mehrere Thioether umfasst, und die an Additionsprodukten reiche Fraktion zurückgewinnen und sie in einer einzigen Leitung zusammenführen.

12. Verwendung einer Anlage nach Anspruch 11, **dadurch gekennzeichnet, dass** sie eine mit den von der ersten und zweiten Trennvorrichtung (5, 10) kommenden Leitungen (6, 12) verbundene Reinigungsvorrichtung (13) umfasst, wobei die Reinigungsvorrichtung (13) dazu fähig ist, ein sekundäres Mercaptan in einer ersten Rohrleitung (16) und ein lineares Mercaptan in einer zweiten Rohrleitung (15) zu isolieren und eine schwere Fraktion, die die Sulfide und andere Nebenprodukte umfasst, in einer dritten Rohrleitung (14) auszutragen.

13. Verwendung einer Anlage nach Anspruch 12, **dadurch gekennzeichnet, dass** sie eine mit dem Ausgang der Reinigungsvorrichtung (13) verbundene Rohrleitung (17) umfasst, die die Sulfide und anderen Nebenprodukte befördert, wobei die Rohrleitung (17) den Fertigstellungsreaktor (8) speist.

## Claims

1. Process for synthesis of a mercaptan starting from a terminal olefin and hydrogen sulfide comprising at least the following successive steps:
- A- catalytic addition of hydrogen sulfide to a terminal olefin catalysed by an acid catalyst, then
- B- separation of the products of the addition reaction into
a light fraction comprising the excess hydrogen sulfide and the olefins, and
a heavy fraction comprising at least one mercaptan and optionally one or more thioethers,
- C- finishing step, passing the light fraction obtained from step B over an acid catalyst, then
- D- separation of the products from the finishing step into
a fraction rich in hydrogen sulfide and
a fraction rich in addition products and then
- E- recycling of the fraction rich in hydrogen sulfide to the step of catalytic addition A.

2. Process according to Claim 1, **characterized in that** the mercaptan synthesized is a secondary mercaptan.

3. Process according to Claim 1 or 2, **characterized in that** the catalyst used in the addition step is a catalyst comprising Lewis acid sites.

4. Process according to any one of the preceding claims, **characterized in that** the addition reaction in step A and/or the finishing step C are heterogeneous catalyses using a solid acid catalyst.

5. Process according to Claim 3 or 4, **characterized in that** the catalyst comprising Lewis acid sites is selected from the oxides of molybdenum, oxides of cobalt, oxides of chromium, cobalt molybdate, phosphotungstic acids deposited on supports selected from activated carbon, alumina, zirconia, silica, thorium oxide, pumice stone and silica-alumina compositions, preferably chromium oxide supported on alumina.

6. Process according to any one of the preceding claims, **characterized in that** the terminal olefin is of the following general formula (1):
R₁R₂C=CH₂ (1),
in which R₁ denotes a hydrogen atom, and
R₂ represents an alkyl radical with from 1 to 20 carbon atoms, preferably from 2 to 20 carbon atoms, and more particularly from 2 to 12 carbon atoms, linear, branched or cyclic.

7. Process according to Claim 6, **characterized in that** the terminal olefin is 1-butene.

8. Process according to any one of the preceding claims, **characterized in that** the catalyst used for the finishing step is an acid catalyst that has strong acid sites, preferably selected from the cation exchange resins and the zeolites, whether exchanged or not.

9. Process according to any one of the preceding claims, **characterized in that** it comprises the following additional step:
- F- purification of the heavy fraction obtained from the separation step B and the fraction rich in addition products obtained from the separation step E, combined into
one or more light fractions comprising one or more mercaptans and
a heavy fraction comprising the sulfides.

10. Process according to any one of the preceding claims, **characterized in that** it comprises the following additional step:
- G- recycling of the heavy fraction from the purification step F defined in Claim 9 to the finishing step C.

11. Use of an installation for carrying out a process for synthesis of a mercaptan starting from a terminal olefin and hydrogen sulfide as defined in any one of the preceding claims comprising:
an addition reactor (1), supplied with hydrogen sulfide and with olefin via pipelines (2, 3),
a pipeline (4), connected to the outlet of the addition reactor (1), supplies a first separating device (5) with a stream of crude reaction mixture,
a pipeline (7), connected to the outlet of the first separating device (5), supplies a finishing reactor (8) with the light fraction comprising the excess hydrogen sulfide and the olefins,
a pipeline (9), connected to the outlet of the finishing reactor (8), supplies a second separating device (10) with all of the reaction mixture from the finishing reactor (8),
a pipeline (11) located at the outlet of the second separating device (10) recycles the fraction rich in hydrogen sulfide to the addition reactor (1) and
pipelines (12, 6, 120, 60) coming from the first and second separating devices (5, 10) recover the heavy fraction comprising at least one mercaptan and optionally one or more thioethers and the fraction rich in addition products, combining them in a single pipeline.

12. Use of an installation according to Claim 11, **characterized in that** it comprises a purification device (13), connected to the pipelines (6, 12) coming from the first and second separating devices (5, 10), the purification device (13) being able to isolate a secondary mercaptan in a first line (16), a linear mercaptan in a second line (15) and to discharge a heavy fraction comprising the sulfides and other by-products in a third line (14).

13. Use of an installation according to Claim 12, **characterized in that** it comprises a line (17), connected to the outlet of the purification device (13), conveying the sulfides and other by-products, the line (17) supplying the finishing reactor (8).
